# FASCICULE DE BREVET EUROPEEN

(11) **EP 4 133 164 B1**
(45) Date de publication et mention de la délivrance du brevet: **21.02.2024**
(21) Numéro de dépôt: 21723888.0
(22) Date de dépôt: 08.04.2021
(51) Int. Cl.: F01M 11/10, F16N 29/04, F01D 25/20, F16K 11/085, F16K 11/16, F02C 7/06, G01N 1/10, G01N 1/20, G01N 15/00, G01N 15/06, G01N 33/28

(54) **DISPOSITIF D'ANALYSE POUR DETECTER DES PARTICULES SOLIDES DANS UN LUBRIFIANT**
ANALYSEVORRICHTUNG ZUM NACHWEIS VON FESTSTOFFPARTIKELN IN EINEM SCHMIERMITTEL
ANALYSIS DEVICE FOR DETECTING SOLID PARTICLES IN A LUBRICANT

(30) Priorité: 08.04.2020 FR 2003537
(43) Date de publication de la demande: 15.02.2023
(73) Titulaire: SAFRAN AIRCRAFT ENGINES, 75015 Paris (FR)
(72) Inventeur: COUPARD, Josselin Xavier, 77550 Moissy-Cramayel (FR); GARNIER, Alméric Pierre Louis, 77550 Moissy-Cramayel (FR); DEMAISON, François Maurice Marcel, 77550 Moissy-Cramayel (FR); LIOTTE, Franck Serge Jacques, 77550 Moissy-Cramayel (FR)
(74) Mandataire: Cabinet Beau de Loménie
(86) Numéro de dépôt international: PCT/FR2021/050625
(87) Numéro de publication internationale: WO 2021/205125

(56) Documents cités:
- EP-A1- 3 363 518
- DE-B3-102007 019 064
- US-A- 3 432 750
- US-A- 4 657 671
- US-A- 5 604 441
- US-A1- 2002 178 780
- US-A1- 2011 214 511
- US-A1- 2015 108 047
- US-A1- 2016 313 237
- US-A1- 2016 370 275

## Description

### Domaine Technique

La présente divulgation concerne le domaine de la surveillance du lubrifiant dans des machines, et plus spécifiquement un dispositif d'analyse pour détecter des particules solides en suspension dans un lubrifiant.

### Technique antérieure

Afin de réduire les coûts d'utilisation, l'on constate dans tout le domaine mécanique une prolongation des délais d'entretien et de renouvellement du lubrifiant. Ainsi, dans le domaine plus spécifique des moteurs à combustion, et notamment des moteurs à turbine à gaz, comme par exemple ceux utilisés dans l'aéronautique, on observe une réduction progressive de la consommation de lubrifiant, qui induit une utilisation plus longue du lubrifiant sans renouvellement.

Avec cette diminution de la fréquence de renouvellement du lubrifiant diminuent aussi les opportunités d'observer et/ou analyser la qualité du lubrifiant lors de vidanges. Or, cette observation et analyse du lubrifiant vidangé permet de détecter non seulement des changements inattendus des propriétés du lubrifiant lui-même, mais aussi, à travers ces propriétés du lubrifiant vidangé, comme par exemple la présence de carburant ou limailles dans le lubrifiant, des dysfonctionnements dans la machine lubrifiée.

Il est déjà connu, voir par exemple la demande de brevet français avec le numéro de publication FR 2 927 401 A1, d'intégrer des capteurs, et notamment des capteurs de particules métalliques, à demeure dans un circuit de lubrification. Les brevets américains US 4 657 671 A, US 5 604 441 A, et US 3 432 750 A divulguaient des dispositifs combinant des capteurs de particules ferromagnétiques et non-ferromagnétiques afin de les détecter séparément.

Toutefois, malgré la présence de tels capteurs, il peut être encore souhaitable de détecter simultanément différents types de particules solides en suspension en les distinguant mieux les unes des autres.

### Exposé de l'invention

Un premier aspect de la présente invention concerne un dispositif d'analyse pour détecter des particules solides en suspension dans un lubrifiant. Le dispositif d'analyse peut comprendre un ou plusieurs capteurs de particules solides ferromagnétiques, et un ou plusieurs autres capteurs aptes à détecter des particules solides non ferromagnétiques. On peut entendre par « particule solide non ferromagnétique », dans ce contexte, toute particule solide présentant une susceptibilité magnétique égale ou inférieure à, par exemple, 10⁴. Ces autres capteurs peuvent être décalés, en direction perpendiculaire à une direction principale d'écoulement du lubrifiant, par rapport aux capteurs de particules solides ferromagnétiques, et le dispositif d'analyse peut comprendre en outre un ou plusieurs aimants disposés de manière à attirer, vers les capteurs de particules solides ferromagnétiques, les particules solides ferromagnétiques en les éloignant des autres capteurs. Les capteurs de particules solides ferromagnétiques peuvent notamment être des capteurs par induction, pouvant comprendre chacun une bobine orientée en direction perpendiculaire à la direction principale d'écoulement du lubrifiant, et les capteurs de particules solides non-ferromagnétiques des capteurs optiques et/ou acoustiques, et en particulier être configurés pour détecter la longueur d'onde et/ou l'intensité de lumière réfléchie par des particules solides non ferromagnétiques. Chacun des capteurs de particules solides ferromagnétiques peut notamment être directionnel et orienté de manière à détecter des particules solides ferromagnétiques en direction perpendiculaire à la direction principale d'écoulement du lubrifiant, et chacun desdits autres capteurs peut notamment être directionnel et orienté de manière à détecter des particules solides non ferromagnétiques en direction parallèle à la direction principale d'écoulement du lubrifiant.

Grâce à cet arrangement, les particules solides ferromagnétiques et non-ferromagnétiques en suspension dans le lubrifiant peuvent être séparément détectées. Cette caractérisation des particules solides en suspension dans le lubrifiant permet en conséquence un meilleur diagnostic de leur origine, ainsi qu'une prévision plus précise des conséquences de cette contamination du lubrifiant.

Suivant un deuxième aspect, le dispositif d'analyse peut comporter aussi une ou plusieurs grilles, disposées transversalement à la direction principale d'écoulement du lubrifiant, pour séparer par taille les particules solides en suspension dans le lubrifiant. Chacun desdits autres capteurs peut alors être disposé de manière à détecter des particules solides non ferromagnétiques sur chacune des grilles. Pour cela chacun desdits autres capteurs peut notamment être disposé en regard d'une grille correspondante parmi lesdites grilles. Les grilles peuvent notamment comprendre au moins une première grille et une deuxième grille disposée en aval de la première grille suivant la direction principale d'écoulement du lubrifiant, la deuxième grille étant plus fine que la première grille, de manière à séparer des particules solides de plus petite taille. On peut obtenir ainsi, à part la séparation entre particules solides ferromagnétiques et non-ferromagnétiques, une séparation par taille, permettant une encore meilleure caractérisation de l'ensemble de particules solides en suspension dans le lubrifiant.

Un troisième aspect de cette divulgation concerne un système de surveillance de lubrifiant comportant le dispositif d'analyse suivant le premier aspect, un ou plusieurs raccords d'admission et un ou plusieurs raccords de sortie. Chacun des raccords d'admission peut être apte à être raccordé, notamment de manière libérable, à un circuit de lubrifiant, de manière à permettre l'admission de lubrifiant, à partir du circuit de lubrifiant, dans les dispositifs d'analyse, et chacun des raccords de sortie est apte à être raccordé, notamment de manière libérable, au circuit de lubrifiant, de manière à permettre le retour du lubrifiant, à travers les dispositifs d'analyse, vers le circuit de lubrifiant.

Grâce à ces caractéristiques, il est possible d'installer ce système de surveillance de lubrifiant sur un circuit de lubrifiant de manière à surveiller de manière continue ou intermittente un ou plusieurs paramètres du lubrifiant pendant une période de fonctionnement du circuit de lubrifiant. Cette installation peut notamment être temporaire.

Quand le système de surveillance de lubrifiant comporte plusieurs desdits raccords d'admission, il peut comporter en outre une valve de sélection d'admission pour mettre lesdits raccords d'admission, de manière sélective, en communication fluide avec les dispositifs d'analyse. Il est ainsi possible de sélectionner alternativement plusieurs points de prélèvement de lubrifiant à surveiller, permettant ainsi l'identification de sources spécifiques de dégradation du lubrifiant dans le circuit.

Par ailleurs, quand le système de surveillance de lubrifiant comporte aussi plusieurs desdits raccords de sortie, il peut aussi comprendre une valve de sélection de sortie pour mettre lesdits raccords de sortie, de manière sélective, en communication fluide avec les dispositifs d'analyse. La valve de sélection d'admission et la valve de sélection de sortie peuvent être couplées, de manière à synchroniser leurs sélections, et ainsi pouvoir retourner le lubrifiant vers la même branche du circuit de lubrification sur laquelle il a été prélevé. La valve de sélection d'admission et/ou la valve de sélection de sortie peuvent comporter un corps de valve rotatif, par exemple en forme de barillet, de manière à permettre la sélection d'un raccord d'admission et/ou d'un raccord de sortie par rotation de ce corps de valve. Toutefois, des formes alternatives de valve de sélection, par exemple à tiroir, peuvent également être considérées.

Afin d'assurer le fonctionnement des capteurs, voire d'autres éléments du système de surveillance de lubrifiant, celui-ci peut comporter aussi un dispositif d'alimentation électrique. Ce dispositif d'alimentation électrique peut notamment comporter une turbine apte à être actionnée par un débit de lubrifiant à travers le système de surveillance de lubrifiant et/ou un thermocouple thermiquement interposé entre le lubrifiant et un puits de chaleur, de manière à pouvoir assurer de manière autonome l'alimentation électrique du système de surveillance de lubrifiant en prélevant de l'énergie thermique ou mécanique du lubrifiant lui-même. Toutefois, il est également envisageable que le dispositif d'alimentation électrique comprenne un dispositif de stockage d'énergie alternativement à ou en combinaison avec lesdits turbine et/ou thermocouple.

Le système de surveillance de lubrifiant peut par ailleurs comporter un dispositif de communication connecté au dispositif d'analyse pour transmettre à un utilisateur et/ou dispositif externe des données captées à travers leurs capteurs.

Par ailleurs, le système de surveillance de lubrifiant peut aussi comprendre un deuxième dispositif d'analyse incluant un ou plusieurs capteurs de qualité du lubrifiant, comme par exemple un capteur optique, un capteur de conductivité électrique, un capteur de température, et/ou un capteur de viscosité.

### Brève description des dessins

L'invention sera bien comprise et ses avantages apparaîtront mieux, à la lecture de la description détaillée qui suit, de modes de réalisation représentés à titre d'exemples non limitatifs. La description se réfère aux dessins annexés sur lesquels :
[Fig. 1] La figure 1 est une illustration schématique partielle d'un circuit de lubrifiant avec un système de surveillance du lubrifiant suivant un mode de réalisation.
[Fig. 2] La figure 2 est une illustration en perspective d'une valve de sélection et une valve de sortie, à corps rotatifs couplés, du système de surveillance de la figure 1.
[Fig. 3A] La figure 3A est une vue en coupe transversale dans le plan III-III de la valve de sélection de la figure 2 en une première position.
[Fig. 3B] La figure 3B est une vue en coupe transversale dans le plan III-III de la valve de sélection de la figure 2 en une deuxième position.
[Fig. 3C] La figure 3C est une vue en coupe transversale dans le plan III-III de la valve de sélection de la figure 2 en une troisième position.
[Fig. 3D] La figure 3D est une vue en coupe transversale dans le plan III-III de la valve de sélection de la figure 2 en une quatrième position.
[Fig. 4] La figure 4 est une illustration schématique d'un dispositif d'analyse appartenant au système de surveillance de la figure 1.

### Description des modes de réalisation

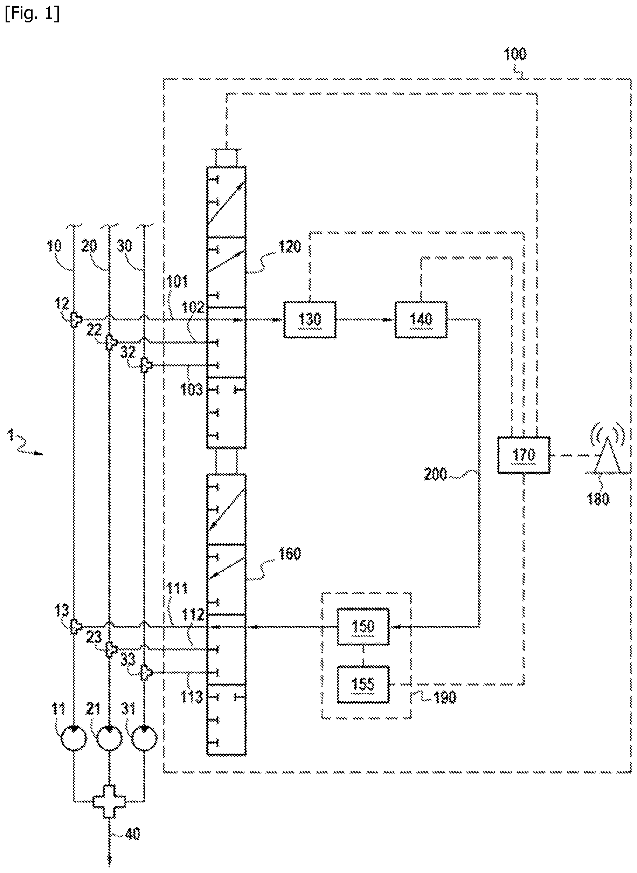

La figure 1 illustre schématiquement une partie d'un circuit de lubrifiant 1 pouvant servir à lubrifier une machine, et notamment une turbomachine telle qu'un turboréacteur d'aéronef. Toutefois, d'autres applications, notamment aéronautiques, automobiles, ferroviaires ou maritimes, sont également envisageables. Comme illustré sur la figure 1, ce circuit de lubrifiant 1 peut comprendre une pluralité de branches 10, 20, 30, qui peuvent provenir de différents organes ou zones de la machine lubrifiée, et se rejoindre, comme illustré, dans un conduit commun 40 en aval de pompes 11, 21, 31 correspondantes. Chacune des branches 10, 20, 30 peut comporter, comme illustré, un branchement amont 12, 22, 32 et un branchement aval 13, 23, 33 permettant son raccordement au système de surveillance du lubrifiant 100 en parallèle aux branches 10, 20, 30. Les branchements amont 12, 22, 32 et aval 13, 23, 33 peuvent être tous disposés en amont des pompes 11, 21, 31 correspondantes. Ils peuvent par exemple substituer les ouvertures conventionnellement utilisées pour l'introduction de capteurs magnétiques de particules métalliques dans le circuit de lubrifiant 1. Des obturateurs non illustrés peuvent obturer ces branchements amont 12, 22, 32 et aval 13, 23, 33 quand le système de surveillance du lubrifiant 100 n'est pas raccordé au circuit de lubrifiant 1.

Le système de surveillance du lubrifiant 100 peut comprendre un raccord d'admission 101, 102, 103 et un raccord de sortie 111, 112, 113 pour chacune des branches 10, 20, 30 du circuit de lubrifiant 1. Chacun des raccords d'admission 101, 102, 103 peut être raccordé, de manière libérable, à un parmi la pluralité de branchements amont 12, 22, 32. De manière analogue, chacun des raccords de sortie 111, 112, 113 peut être raccordé, de manière libérable, à un parmi la pluralité des branchements aval 13,23,33.

Bien que, dans l'exemple illustré, le circuit de lubrifiant 1 compte trois branches 10, 20, 30 et le système de surveillance du lubrifiant 100 compte donc le même nombre de raccords d'admission 101, 102, 103 et de raccords de sortie 111, 112, 113, il est envisageable d'avoir un nombre différent de branches et donc de raccords d'admission et de sortie correspondants. Il est également envisageable d'avoir un plus grand nombre de raccords d'admission que de raccords de sortie, si par exemple au moins certaines des branches se rejoignent à une confluence en amont des pompes, de telle manière qu'un raccord de sortie du système de surveillance du lubrifiant puisse être raccordé au circuit de lubrifiant entre la confluence et la pompe en aval, ou si un unique raccord de sortie est raccordé au conduit commun 40 en aval des pompes 11, 21, 31, ce qui nécessiterait d'autre part d'intégrer aussi une pompe au système de surveillance du lubrifiant 100.

Entre les raccords d'admission 101, 102, 103 et les raccords de sortie 111, 112, 113, le système de surveillance du lubrifiant 100 peut comporter une valve de sélection d'admission 120, un premier dispositif d'analyse 130, un deuxième dispositif d'analyse 140, un dispositif de prélèvement énergétique 150, et une valve de sélection de sortie 160 en communication fluide en série. Par ailleurs, le système de surveillance du lubrifiant 100 peut comporter une unité de commande 170, un dispositif de communication 180, et un dispositif d'alimentation électrique 190 dont peut faire partie le dispositif de prélèvement énergétique 150.

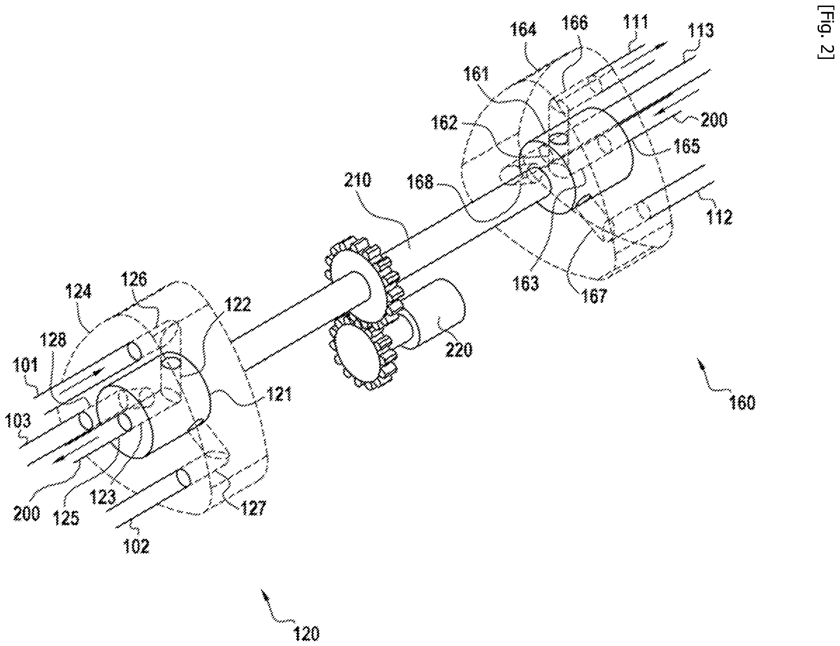

La valve de sélection d'admission 120 peut être configurée de manière à mettre sélectivement en communication fluide chacun des raccords d'admission 101, 102, 103 avec un conduit 200 traversant les dispositifs d'analyse 130, 140, voire même le dispositif de prélèvement énergétique 150, en aval. La valve de sélection d'admission 120 peut aussi être configurée pour isoler le conduit 200 de l'ensemble des raccords d'admission 101, 102, 103, de manière à ce que le lubrifiant puisse continuer à circuler sur chacune des branches 10, 20, 30 du circuit de lubrifiant 1 sans être prélevé. De manière analogue, la valve de sélection de sortie 160 peut être configurée de manière à mettre le conduit 200, en aval des dispositifs d'analyse, voire en aval même du dispositif de prélèvement énergétique 150, sélectivement en communication fluide avec chacun des raccords de sortie 111, 112, 113, ou à l'isoler de l'ensemble des raccords de sortie 111, 112, 113. Les valves de sélection d'admission 120 et de sortie 160 peuvent notamment prendre la forme de valves à corps rotatif, de préférence couplées, par exemple mécaniquement comme illustré sur la figure 2.

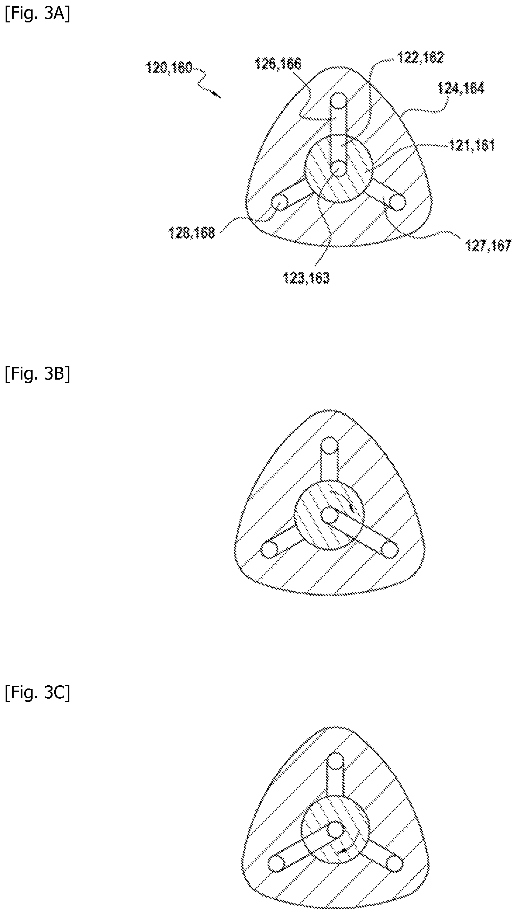

Comme on peut voir sur les figures 2 et 3A à 3C, les corps de valve rotatifs 121, 161 de, respectivement, la valve de sélection d'admission 120 et la valve de sélection de sortie 160, peuvent comprendre chacun un perçage radial 122, 162 et un perçage axial 123, 163, en communication fluide l'un avec l'autre. Le perçage radial 122, 162 peut déboucher sur une surface périphérique du corps de valve rotatif 121, 161, tandis que le perçage axial 123,163 peut déboucher sur une surface frontale du corps de valve rotatif 121, 161. La valve de sélection d'admission 120 et la valve de sélection de sortie 160 peuvent aussi comprendre chacune un carter de valve 124,164 avec un orifice central 125, 165, qui peut être en regard du perçage axial 123, 163, et des orifices périphériques 126, 127,128, 166, 167, 168.

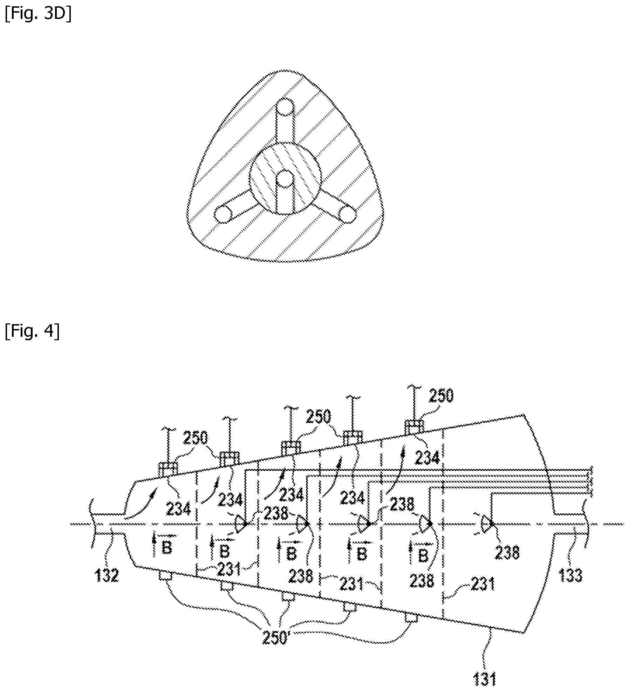

Les orifices radiaux 126, 127, 128 du carter de valve 124 de la valve de sélection d'admission 120 peuvent être chacun en communication fluide avec l'un des raccords d'admission 101,102, 103. L'orifice central 125 du carter de valve 124 peut être en communication fluide avec le conduit 200. Le perçage radial 122 du corps de valve rotatif 121 de la valve de sélection d'admission 120 peut être sélectivement mis en regard de chacun de ces orifices périphériques 126, 127, 128 par rotation relative du corps de valve rotatif 121 par rapport au carter de valve 124 autour de son axe central X, de manière à mettre sélectivement en communication fluide chacun des raccords d'admission 101, 102, 103 avec le conduit 200, comme illustré sur les figures 3A à 3C. Le perçage radial 122 du corps de valve rotatif 121 de la valve de sélection d'admission 120 peut également tourner vers une position intermédiaire, comme illustré sur la figure 3D, de manière à isoler le conduit 200 des trois raccords d'admission 101, 102, 103.

De manière analogue, les orifices radiaux 166, 167, 168 du carter de valve 164 de la valve de sélection de sortie 160 peuvent être chacun en communication fluide avec l'un des raccords de sortie 111,112, 113. L'orifice central 165 du carter de valve 164 peut être en communication fluide avec le conduit 200. Le perçage radial 162 du corps de valve rotatif 161 de la valve de sélection de sortie 160 peut être sélectivement mis en regard de chacun de ces orifices périphériques 166, 167, 168 par rotation relative du corps de valve rotatif 161 par rapport au carter de valve 164 autour de son axe central X, de manière à mettre sélectivement en communication fluide chacun des raccords de sortie 111, 112, 113 avec le conduit 200, ou à isoler ce conduit 200 des trois raccords de sortie 111, 112, 113.

Comme illustré sur la figure 2, les corps de valve rotatifs 121, 161 peuvent être couplés mécaniquement par un arbre rotatif commun 210, qui peut être couplé mécaniquement à son tour à un dispositif d'actionnement 220 tel que, par exemple, un moteur pas-à-pas connecté électriquement à l'unité de commande 170 pour son alimentation électrique et sa commande. Ainsi, la valve de sélection d'admission 120 et la valve de sélection de sortie 160 peuvent être synchronisées dans leurs sélections respectives. Toutefois, la valve de sélection d'admission 120 et/ou la valve d'admission de sortie 160 peuvent avoir une configuration différente à la configuration rotative illustrée, et prendre par exemple la forme de valves à tiroirs.

Comme illustré sur la figure 4, le premier dispositif d'analyse 130 peut être un dispositif d'analyse de particules solides en suspension dans le lubrifiant. Il peut comprendre un carter 131 avec une succession de grilles 231 de plus en plus fines entre son admission 132 et sa sortie 133, de manière à séparer les particules solides par taille. Par exemple, une première grille 231 peut avoir une ouverture de maille de 8 mm², une deuxième grille 231, en aval de la première grille 231, peut avoir une ouverture de maille de 4 mm², une troisième grille 231, en aval de la deuxième grille 231, peut avoir une ouverture de maille de 1 mm², une quatrième grille 231, en aval de la troisième grille 231, peut avoir une ouverture de maille de 0,1 mm², et une cinquième grille 231, en aval de la quatrième grille 231, peut avoir une ouverture de maille de 0,02 mm², de manière chacune à arrêter les particules de section supérieure à l'ouverture de maille correspondante. L'ouverture de maille des grilles successives pourrait alternativement suivre une progression inverse carrée, suivant la formule Aᵢ=A₁/i², où i est la position de la grille d'amont en aval et Aᵢ l'ouverture de maille de la grille respective. Des moyens alternatifs de séparation des particules solides par taille, par exemple par centrifugation, sont aussi envisageables.

Le premier dispositif d'analyse 130 peut aussi comprendre des capteurs 234 de particules ferromagnétiques, situés par exemple affleurant chacun sur les parois du carter 131, en amont de chaque grille 231, pour détecter les particules ferromagnétiques de différentes tailles correspondantes. Ces capteurs 234 de particules ferromagnétiques peuvent être, par exemple, des capteurs inductifs, et notamment comprendre chacun une bobine (non illustrée), dont l'axe peut être orienté perpendiculairement à la direction principale d'écoulement du lubrifiant entre l'admission 132 et la sortie 133 du premier dispositif d'analyse 130, de manière à détecter les particules ferromagnétiques dans l'axe de la bobine par variation d'un champ magnétique traversant la bobine. Ainsi, chaque capteur 234 peut être configuré pour détecter, avec par exemple au moins 65% d'efficacité, une particule ferromagnétique de, par exemple, au moins 0,130 mg, avec un rapport de taille longueur-largeur de jusqu'à, par exemple, 20:1.

Un ou plusieurs aimants 250 peuvent être disposés en périphérie du carter 131 pour attirer les particules ferromagnétiques vers les capteurs 234 de particules ferromagnétiques. Chacun de ces aimants 250 peut notamment être disposé coaxial à la bobine d'un des capteurs 234 de particules ferromagnétiques, de manière non seulement à attirer les particules ferromagnétiques vers le capteur 234 correspondant, mais aussi à fournir le champ magnétique dont la variation permettra de les détecter par le capteur 234. Chaque aimant 250 peut être un aimant permanent ou, alternativement, un électroaimant avec une bobine qui peut alors être coaxiale à celle du capteur 234 correspondant. Eventuellement, un autre aimant 250', orienté suivant la même polarité, peut être disposé en regard de chacun des aimants 250 sur une paroi opposée du carter 230. Les aimants 250 peuvent être d'intensités différentes, et notamment d'intensités croissantes dans le sens d'écoulement du lubrifiant de l'admission 132 à la sortie 133 du premier dispositif d'analyse 130, pour compenser la taille décroissante des particules solides ferromagnétiques traversant les grilles 231 successives. Pour obtenir ces différentes intensités, ils peuvent par exemple être des électroaimants avec des bobines ayant des différents nombres de spires, alimentées avec un même voltage. Il est aussi envisageable de piloter le voltage d'alimentation de chaque électroaimant, conjointement ou séparément, et notamment en fonction du débit de lubrifiant traversant le premier dispositif d'analyse 130. Le voltage d'alimentation de chaque électroaimant peut être par exemple entre 0 et 24 V, et de préférence entre 120 mV et 4V.

Le premier dispositif d'analyse 130 peut comprendre aussi d'autres capteurs de particules 238 qui peuvent être disposés, par exemple dans une zone centrale du carter 131, en regard de chaque grille 231, pour détecter les particules non ferromagnétiques des différentes tailles correspondantes prises dans chaque grille 231. Ces autres capteurs de particules 238 peuvent être notamment des capteurs acoustiques et/ou optiques. En particulier, ils peuvent être des capteurs optiques pour réaliser de la réflectométrie, configurés pour détecter une longueur d'onde et/ou intensité de lumière réfléchie par les particules solides non ferromagnétiques, et ainsi permettre de distinguer entre différents types de particules solides non ferromagnétiques. La lumière réfléchie par les particules solides non ferromagnétiques peut provenir d'une ou plusieurs sources, par exemple des diodes électroluminescentes, intégrées dans chaque capteur 238, ou externes à ceux-ci. Afin de permettre d'illuminer les particules solides non ferromagnétiques à travers le lubrifiant, la lumière émise par ces sources et captée par les capteurs 238 peut être restreinte à certaines bandes spectrales, en particulier dans le spectre visible. Des capteurs de contrainte (non illustrés) peuvent aussi être couplés à chaque grille 231, et contribuer à mesurer la quantité de particules solides sur chacune des grilles 231.

Grâce au décalage, des capteurs 234 de particules ferromagnétiques, perpendiculaire à la direction principale d'écoulement du lubrifiant, qui peuvent être disposés en périphérie du carter 131, par rapport aux autres capteurs 238, situés dans la zone centrale du carter 131, et à la disposition des aimants 250 pour attirer les particules ferromagnétiques vers les capteurs 234 en les éloignant des autres capteurs 238, il est possible d'éviter la détection de particules solides ferromagnétiques par ces autres capteurs 238, pour ainsi détecter séparément les deux types de particules solides. Chacun des capteurs 234 et 238 peut être connecté à l'unité de commande 170 pour lui transmettre la détection de ces particules.

Le deuxième dispositif d'analyse 140 peut comprendre un ou plusieurs autres capteurs tels que, par exemple, un capteur de conductivité électrique, un capteur optique pour capter la couleur et/ou la turbidité du lubrifiant, un capteur de viscosité, un thermomètre, un manomètre, un capteur de vibration et/ou un capteur acoustique. Chacun de ces capteurs du deuxième dispositif d'analyse peut également être connecté à l'unité de commande 170 pour lui transmettre les données captées par ceux-ci.

Le dispositif de prélèvement énergétique 150 peut être une turbine apte à être impulsée par le débit de lubrifiant à travers le conduit 200 et couplée à un générateur électrique, ou un thermocouple apte à produire de l'électricité à partir du gradient thermique entre le lubrifiant circulant à travers le conduit 200 et un puits de chaleur tel que, par exemple, un radiateur, une veine d'air et/ou un circuit de carburant. Ce dispositif de prélèvement énergétique 150 peut être connecté électriquement à un dispositif de stockage énergétique 155, qui peut être par exemple une batterie, un condensateur et/ou un volant d'inertie, pour former le dispositif d'alimentation électrique 190. Ce dispositif d'alimentation électrique 190 peut être connecté électriquement aux autres organes du système de surveillance du lubrifiant 100 de manière à en assurer l'alimentation électrique, éventuellement même de manière autonome.

Toutefois, alternativement ou en complément à ce dispositif d'alimentation électrique 190, on peut également envisager une connexion électrique externe. Par ailleurs, il est aussi envisageable que le dispositif d'alimentation électrique 190 ne comporte pas de dispositif de prélèvement énergétique, et assure l'alimentation électrique du système de surveillance du lubrifiant 100 à partir uniquement de l'énergie préalablement stockée dans le dispositif de stockage énergétique 155, ou alternativement qu'il ne comporte pas de dispositif de stockage énergétique et assure l'alimentation électrique du système de surveillance du lubrifiant à partir de l'énergie prélevée par le dispositif de prélèvement énergétique 150 sur le débit de lubrifiant le traversant.

L'unité de commande 170 peut être un calculateur électronique, éventuellement programmable. Ainsi, elle peut être intégrée dans un circuit intégré ou microprocesseur, et incorporer un organe de stockage de données. Finalement, l'unité de commande 170 est connectée à un dispositif de communication 180 pour transmettre à des systèmes externes et/ou à des utilisateurs les données captées par les différents capteurs des dispositifs d'analyse 130, 140, et/ou les résultats de leur analyse par l'unité de commande 170. Ce dispositif de communication 180 peut être, comme illustré, un dispositif de communication sans fil, mais également un simple connecteur électrique et/ou optique pour la transmission de données. L'unité de commande 170 peut aussi être configurée pour mettre le système de surveillance du lubrifiant 100 en mode inactif, notamment en actionnant les valves de sélection d'admission 120 et de sortie 160 pour isoler le conduit 200 des raccords d'admission 101, 102, 103, et de sortie 111, 112, 113, par exemple au cas où le dispositif d'alimentation électrique 190 ne pourrait plus fournir une puissance électrique suffisante pour le fonctionnement normal du système de surveillance du lubrifiant 100.

Pour utiliser le système de surveillance du lubrifiant 100, on peut procéder d'abord à son installation en raccordant un raccord d'admission 101, 102, 103 et un raccord de sortie 111, 112, 113 du système de surveillance du lubrifiant 100 à chacune des branches 10, 20, 30 du circuit de lubrifiant 1 à travers le branchement amont 12, 22, 32 et le branchement aval 13, 23, 33 respectifs, après en avoir retiré les obturateurs.

Dans une étape subséquente de surveillance du lubrifiant, du lubrifiant peut être successivement dérivé de chacune des branches 10, 20, 30 à travers le conduit 200 du système de surveillance du lubrifiant 100 en sélectionnant chaque raccord d'admission 101, 102, 103 et raccord de sortie 111, 112, 113 correspondant avec la valve de sélection d'admission 120 et la valve d'admission de sortie 160, éventuellement commandées par l'unité de commande 170. Le lubrifiant circulant à travers le conduit 200 peut alors traverser le premier dispositif d'analyse 130, dont les différents capteurs pourront détecter séparément les particules solides métalliques et non métalliques de différentes tailles, et le deuxième dispositif d'analyse 140, dont les différents capteurs pourront détecter des propriétés du lubrifiant telles que par exemple sa couleur, turbidité, conductivité électrique, viscosité, température et/ou pression, ainsi que d'éventuels sons et/ou vibrations. Les données captées par les capteurs des dispositifs d'analyse 130, 140 pendant cette étape de surveillance peuvent être transmises à l'unité de commande 170 pour leur traitement, analyse, stockage et/ou transmission à travers le dispositif de communication 180. En outre, pendant cette étape de surveillance, le dispositif d'alimentation électrique 190 peut alimenter électriquement les différents autres organes du système de surveillance du lubrifiant 100 avec de l'énergie électrique prélevée à travers le dispositif de prélèvement énergétique 150 sur le débit de lubrifiant le traversant et/ou récupérée du dispositif de stockage énergétique 155.

Lorsqu'il est décidé de finaliser la surveillance du lubrifiant, le système de surveillance du lubrifiant 100 peut être désinstallé en séparant chaque raccord d'admission 101, 102, 103 et chaque raccord de sortie 111, 112, 113 du système de surveillance du lubrifiant 100 des branchements amont 12, 22, 32 et des branchements aval 13, 23, 33 respectifs, et en refermant ces derniers avec les obturateurs.

Quoique la présente invention ait été décrite en se référant à des exemples de réalisation spécifiques, il est évident que des différentes modifications et changements peuvent être effectués sur ces exemples sans sortir de la portée générale de l'invention telle que définie par les revendications. En outre, des caractéristiques individuelles des différents modes de réalisation évoqués peuvent être combinées dans des modes de réalisation additionnels. Par conséquent, la description et les dessins doivent être considérés dans un sens illustratif plutôt que restrictif.

## Revendications

1. Dispositif d'analyse (130) pour détecter des particules solides en suspension dans un lubrifiant, le dispositif d'analyse (100) comprenant :
un ou plusieurs capteurs de particules solides ferromagnétiques (234),
un ou plusieurs autres capteurs (238) aptes à détecter des particules solides non ferromagnétiques, ces autres capteurs (238) étant décalés, en direction perpendiculaire à une direction principale d'écoulement du lubrifiant, par rapport aux capteurs de particules solides ferromagnétiques (234), et
un ou plusieurs aimants (250) disposés de manière à attirer, vers les capteurs de particules solides ferromagnétiques (234), les particules solides ferromagnétiques en les éloignant des autres capteurs (238).

2. Dispositif d'analyse (130) suivant la revendication 1, dans lequel chacun des capteurs de particules solides ferromagnétiques (234) est directionnel et orienté de manière à détecter des particules solides ferromagnétiques en direction perpendiculaire à la direction principale d'écoulement du lubrifiant.

3. Dispositif d'analyse (130) suivant l'une quelconque des revendications 1 ou 2, dans lequel chacun des autres capteurs (238) est directionnel et orienté de manière à détecter des particules solides non ferromagnétiques en direction parallèle à la direction principale d'écoulement du lubrifiant.

4. Dispositif d'analyse (130) suivant l'une quelconque des revendications 1 à 3, comportant aussi une ou plusieurs grilles (231), disposées transversalement à la direction principale d'écoulement du lubrifiant, pour séparer par taille les particules solides en suspension dans le lubrifiant.

5. Dispositif d'analyse (130) suivant la revendication 4, dans lequel chacun desdits autres capteurs (238) est disposé de manière à détecter des particules solides non ferromagnétiques sur chacune des grilles (231).

6. Dispositif d'analyse (130) suivant l'une quelconque des revendications 4 ou 5, dans lequel les grilles comprennent au moins une première grille (231) et une deuxième grille (231) disposée en aval de la première grille (231) suivant la direction principale d'écoulement du lubrifiant, la deuxième grille (231) étant plus fine que la première grille (231), de manière à séparer des particules solides de plus petite taille.

7. Dispositif d'analyse (130) suivant l'une quelconque des revendications 1 à 6, dans lequel les capteurs de particules solides ferromagnétiques (234) sont des capteurs par induction.

8. Dispositif d'analyse (130) suivant la revendication 7, dans lequel chaque capteur de particules solides ferromagnétiques (234) comprend une bobine orientée en direction perpendiculaire à la direction principale d'écoulement du lubrifiant.

9. Dispositif d'analyse (130) suivant l'une quelconque des revendications 1 à 8, dans lequel les autres capteurs (238) sont des capteurs optiques et/ou acoustiques.

10. Dispositif d'analyse (130) suivant la revendication 9, dans lequel chacun desdits autres capteurs (238) est configuré pour détecter une longueur d'onde et/ou intensité de lumière réfléchie par des particules solides non ferromagnétiques.

11. Système (100) de surveillance de lubrifiant comportant :
le dispositif d'analyse (130) suivant l'une quelconque des revendications 1 à 10,
un ou plusieurs raccords d'admission (101,102,103), chacun apte à être raccordé à un circuit de lubrifiant (1), de manière à permettre l'admission de lubrifiant, à partir du circuit de lubrifiant (1), dans le dispositif d'analyse (130),
un ou plusieurs raccords de sortie (111,112,113), chacun apte à être raccordé au circuit de lubrifiant (1), de manière à permettre le retour du lubrifiant, à travers le dispositif d'analyse (130), vers le circuit de lubrifiant (1).

12. Système (100) de surveillance de lubrifiant suivant la revendication 11, dans lequel les raccords d'admission (101,102,103) et les raccords de sortie (111,112,113) sont aptes à être raccordés de manière libérable au circuit de lubrifiant (1).

13. Système (100) de surveillance de lubrifiant suivant l'une quelconque des revendication 11 ou 12, comportant plusieurs desdits raccords d'admission (101,102,103), et une valve de sélection d'admission (120) pour mettre lesdits raccords d'admission (101,102,103), de manière sélective, en communication fluide avec les dispositifs d'analyse (130,140).

14. Système (100) de surveillance de lubrifiant suivant la revendication 13, dans lequel la valve de sélection d'admission (120) comporte un corps de valve rotatif (121).

15. Système (100) de surveillance de lubrifiant suivant l'une quelconque des revendications 13 ou 14, comportant plusieurs desdits raccords de sortie (111,112,113), et une valve de sélection de sortie (160) pour mettre lesdits raccords de sortie (111,112,113), de manière sélective, en communication fluide avec le dispositif d'analyse (130).

16. Système (100) de surveillance de lubrifiant suivant la revendication 15, dans lequel la valve de sélection d'admission (120) et la valve de sélection de sortie (160) sont couplées.

17. Système (100) de surveillance de lubrifiant suivant l'une quelconque des revendications 11 à 16, comportant un dispositif de communication (180) connecté au dispositif d'analyse (130).

## Patentansprüche

1. Analysevorrichtung (130) zum Nachweis von Feststoffpartikeln in Suspension in einem Schmierstoff, wobei die Analysevorrichtung (100) umfasst:
einen oder mehrere Sensoren (234) für ferromagnetische Feststoffpartikel,
einen oder mehrere andere Sensoren (238), die dazu geeignet sind, nicht ferromagnetische Feststoffpartikel nachzuweisen, wobei diese anderen Sensoren (238) in der Richtung senkrecht zu einer Hauptströmungsrichtung des Schmierstoffs in Bezug auf die Sensoren (234) für ferromagnetische Feststoffpartikel versetzt sind, und
einen oder mehrere Magneten (250), die derart angeordnet sind, dass die ferromagnetischen Feststoffpartikel hin zu den Sensoren (234) für ferromagnetische Feststoffpartikel angezogen und dabei von den anderen Sensoren (238) entfernt werden.

2. Analysevorrichtung (130) nach Anspruch 1, wobei jeder der Sensoren (234) für ferromagnetische Feststoffpartikel derart gerichtet und ausgerichtet ist, dass er ferromagnetische Feststoffpartikel in der Richtung senkrecht zur Hauptströmungsrichtung des Schmierstoffs nachweist.

3. Analysevorrichtung (130) nach einem der Ansprüche 1 oder 2, wobei jeder der anderen Sensoren (238) derart gerichtet und ausgerichtet ist, dass er nicht ferromagnetische Feststoffpartikel in der Richtung parallel zur Hauptströmungsrichtung des Schmierstoffs nachweist.

4. Analysevorrichtung (130) nach einem der Ansprüche 1 bis 3, die auch ein oder mehrere Gitter (231) beinhaltet, die quer zur Hauptströmungsrichtung des Schmierstoffs angerordnet sind, um die Feststoffpartikel in Suspension in dem Schmierstoff nach Größe zu trennen.

5. Analysevorrichtung (130) nach Anspruch 4, wobei jeder der anderen Sensoren (238) derart angeordnet ist, dass er nicht ferromagnetische Feststoffpartikel an jedem der Gitter (231) nachweist.

6. Analysevorrichtung (130) nach einem der Ansprüche 4 oder 5, wobei die Gitter mindestens ein erstes Gitter (231) und ein zweites Gitter (231) umfassen, das stromabwärts des ersten Gitters (231) entlang der Hauptströmungsrichtung des Schmierstoffs angeordnet ist, wobei das zweite Gitter (231) feiner ist als das erste Gitter (231), derart dass Feststoffpartikel mit kleinerer Größe getrennt werden.

7. Analysevorrichtung (130) nach einem der Ansprüche 1 bis 6, wobei die Sensoren (234) für ferromagnetische Feststoffpartikel Induktionssensoren sind.

8. Analysevorrichtung (130) nach Anspruch 7, wobei jeder Sensor (234) für ferromagnetische Feststoffpartikel eine Spule umfasst, die in der Richtung senkrecht zur Hauptströmungsrichtung des Schmierstoffs ausgerichtet ist.

9. Analysevorrichtung (130) nach einem der Ansprüche 1 bis 8, wobei die anderen Sensoren (238) optische und/oder akustische Sensoren sind.

10. Analysevorrichtung (130) nach Anspruch 9, wobei jeder der anderen Sensoren (238) dazu ausgestaltet ist, eine Wellenlänge und/oder Stärke von Licht nachzuweisen, das von den nicht ferromagnetischen Feststoffpartikeln reflektiert wird.

11. System (100) zur Schmierstoffüberwachung, das beinhaltet:
die Analysevorrichtung (130) nach einem der Ansprüche 1 bis 10,
einen oder mehrere Einlassanschlüsse (101, 102, 103), der bzw. die jeweils dazu geeignet ist bzw. sind, an einen Schmierstoffkreis (1) angeschlossen zu werden, um den Einlass von Schmierstoff ausgehend von dem Schmierstoffkreis (1) in die Analysevorrichtung (130) zu erlauben,
einen oder mehrere Ausgangsanschlüsse (111, 112, 113), der bzw. die jeweils dazu geeignet ist bzw. sind, an den Schmierstoffkreis (1) angeschlossen zu werden, um die Rückkehr des Schmierstoffs durch die Analysevorrichtung (130) hin zu dem Schmierstoffkreis (1) zu erlauben.

12. System (100) zur Schmierstoffüberwachung nach Anspruch 11, wobei die Einlassanschlüsse (101, 102, 103) und die Ausgangsanschlüsse (111, 112, 113) dazu geeignet sind, lösbar an den Schmierstoffkreis (1) angeschlossen zu werden.

13. System (100) zur Schmierstoffüberwachung nach einem von Anspruch 11 oder 12, das mehrere von den Einlassanschlüssen (101, 102, 103) und ein Einlassauswahlventil (120) umfasst, um die Einlassanschlüsse (101, 102, 103) selektiv mit den Analysevorrichtungen (130, 140) in Fluidverbindung zu bringen.

14. System (100) zur Schmierstoffüberwachung nach Anspruch 13, wobei das Einlassauswahlventil (120) einen drehbaren Ventilkörper (121) beinhaltet.

15. System (100) zur Schmierstoffüberwachung nach einem der Ansprüche 13 oder 14, das mehrere von den Ausgangsanschlüssen (111, 112, 113) und ein Ausgangsauswahlventil (160) umfasst, um die Ausgangsanschlüsse (111, 112, 113) selektiv mit der Analysevorrichtung (130) in Fluidverbindung zu bringen.

16. System (100) zur Schmierstoffüberwachung nach Anspruch 15, wobei das Einlassauswahlventil (120) und das Ausgangsauswahlventil (160) gekoppelt sind.

17. System (100) zur Schmierstoffüberwachung nach einem der Ansprüche 11 bis 16, das eine mit der Analysevorrichtung (130) verbundene Kommunikationsvorrichtung (180) beinhaltet.

## Claims

1. An analysis device (130) for detecting solid particles in suspension in a lubricant, the analysis device (100) comprising:
one or more sensors (234) of ferromagnetic solid particles, one or more other sensors (238) able to detect non-ferromagnetic solid particles, these other sensors (238) being offset in a direction perpendicular to a main direction of flow of the lubricant in relation to the sensors (234) of ferromagnetic solid particles, and one or more magnets (250) arranged so as to attract the ferromagnetic solid particles towards the sensors (234) of ferromagnetic solid particles by drawing them away from the other sensors (238).

2. The analysis device (130) according to claim 1, wherein each of the sensors (234) of ferromagnetic solid particles is directional and oriented to detect ferromagnetic solid particles in a direction perpendicular to the main direction of flow of the lubricant.

3. The analysis device (130) according to any of claims 1 or 2, wherein each of the other sensors (238) is directional and oriented to detect non-ferromagnetic solid particles in a direction parallel to the main direction of flow of the lubricant.

4. The analysis device (130) according to any of claims 1 to 3, further comprising one or more screens (231) arranged crosswise to the main direction of flow of the lubricant, to separate per size the solid particles in suspension in the lubricant.

5. The analysis device (130) according to claim 4, wherein each of said other sensors (238) is arranged to detect non-ferromagnetic solid particles on each of the screens (231).

6. The analysis device (130) according to any of claims 4 or 5, wherein the screens comprise at least one first screen (231) and a second screen (231) arranged downstream of the first screen (231) in the main direction of flow of the lubricant, the second screen (231) being finer than the first screen (231) so as to separate solid particles of smaller size.

7. The analysis device (130) according to any of claims 1 to 6, wherein the sensors (234) of ferromagnetic solid particles are inductive sensors.

8. The analysis device (130) according to claim 7, wherein each sensor (234) of ferromagnetic solid particles comprises a winding oriented in a direction perpendicular to the main direction of flow of the lubricant.

9. The analysis device (130) according to any of claims 1 to 8, wherein the other sensors (238) are optical and/or acoustic sensors.

10. The analysis device (130) according to claim 9, wherein each of said other sensors (238) is configured to detect a wavelength and/or light intensity reflected by non-ferromagnetic solid particles.

11. A lubricant monitoring system (100) comprising: the analysis device (130) according to any of claims 1 to 10,
one or more inlet connections (101, 102, 103) each able to be connected to a lubricant circuit (1) to allow the entry of lubricant from the lubricant circuit (1) into the analysis device (130),
one or more outlet connections (111, 112, 113) each able to be connected to the lubricant circuit (1) so as to allow return of the lubricant through the analysis device (130) towards the lubricant circuit (1).

12. The lubricant monitoring system (100) according to claim 11, wherein the inlet connections (101, 102, 103) and outlet connections (111, 112, 113) are able to be connected releasably to the lubricant circuit (1).

13. The lubricant monitoring system (100) according to any of claims 11 or 12, comprising several said inlet connections (101, 102, 103) and a selective inlet valve (120) to place said inlet connections (101, 102, 103) selectively in fluid communication with the analysis devices (130, 140).

14. The lubricant monitoring system (100) according to claim 13, wherein the selective inlet valve (120) comprises a rotative valve body (121).

15. The lubricant monitoring system (100) according to any of claims 13 or 14, comprising several said outlet connections (111, 112, 113) and a selective outlet valve (160) to place said outlet connections (111, 112, 113) selectively in fluid communication with the analysis device (130).

16. The lubricant monitoring system (100) according to claim 15, wherein the selective inlet valve (120) and selective outlet valve (160) are coupled together.

17. The lubricant monitoring system (100) according to any of claims 11 to 16, comprising a communication device (180) connected to the analysis device (130).
